# FASCICULE DE BREVET EUROPEEN

(11) **EP 2 385 046 B1**
(45) Date de publication et mention de la délivrance du brevet: **19.09.2012**
(21) Numéro de dépôt: 11164323.5
(22) Date de dépôt: 29.04.2011
(51) Int. Cl.: C07D 401/12

(54) **Phase précurseur et son utilisation pour préparer le sel de magnésium tétrahydraté d'un énantiomère d'oméprazole**
Ausgangsphase und ihre Verwendung zur Herstellung des Tetrahydrat-Magnesiumsalzes eines Enantiomers vom Omeprazol
Precursor phase and use thereof for preparing the magnesium salt tetrahydrate of an omeprazole enantiomer

(30) Priorité: 03.05.2010 FR 1053419
(43) Date de publication de la demande: 09.11.2011
(73) Titulaire: PRODUITS CHIMIQUES AUXILIAIRES ET DE SYNTHESE, F-91160 Longjumeau (FR); UNIVERSITE DE ROUEN, 76130 Mont-Saint-Aignan (FR)
(72) Inventeur: Chouippe, Snella, 28500 Charpont (FR); Schneider, Jean-Marie, 78200 Magnanville (FR); Tauvel, Guillaume, 76000 Rouen (FR); Coquerel, Gérard, 76130 Mont Saint Aignan (FR)
(74) Mandataire: Cabinet Plasseraud

(56) Documents cités:
- WO-A1-01/14367
- FR-A1- 2 920 428

## Description

La présente invention concerne une phase précurseur du sel de magnésium tétrahydraté d'un énantiomère d'oméprazole, ainsi que ses procédés de préparation et son utilisation pour la préparation dudit sel de magnésium tétrahydraté. Elle concerne également les cristaux du sel de magnésium tétrahydraté ainsi obtenu, ainsi que leur utilisation, notamment dans la préparation du dihydrate forme A du sel de magnésium dudit énantiomère.

Les sels alcalins et alcalino-terreux de l'oméprazole et de ses énantiomères sont bien connus comme inhibiteurs de sécrétion gastrique et agents anti-ulcéreux. Parmi ceux-ci, le sel de magnésium de l'énantiomère (S) de l'oméprazole, ou esoméprazole ou 5-méthoxy-2-[(S)-[4-méthoxy-3,5-diméthyl-2-pyridinyl) méthyl] sulfinyl]-1H-benzimidazole, est le premier inhibiteur de la pompe à protons (IPP) développé et commercialisé sous forme d'énantiomère pur. La société ASTRA ZENECA exploite ainsi ce composé sous forme de trihydrate, sous la dénomination commerciale Inexium^{®}. D'autres sociétés ont développé un sel de magnésium d'esoméprazole sous forme de dihydrate.

Des procédés de préparation de ces composés sont décrits dans la littérature.

Ainsi, la demande de brevet WO 2004/002982 décrit la séparation de l'oméprazole racémique en ses énantiomères purs par formation de sels diastéréomères à partir du sel de sodium de l'Oméprazole racémique mis en présence de l'agent de coordination diéthyl-D-Tartrate/Ti(iso-Pr)₄ dans l'acétone et complexation à l'aide de l'acide L-mandélique. La cristallisation sélective du diastéréomère comprenant l'esoméprazole suivie d'une hydrolyse basique donne le sel de magnésium d'esoméprazole trihydrate de 99% ee. La formation du dihydrate correspondant est obtenue par séchage contrôlé.

La demande de brevet WO 2004/046134 décrit la préparation du sel de magnésium de l'esoméprazole trihydrate de forme cristalline II à partir de la forme amorphe du même sel, qui est dissoute dans le méthanol pour former une solution. Après filtration, élimination du solvant et précipitation dans l'eau, on obtient un solide qui est lavé avec un mélange d'eau et d'acétone avant d'être séché.

La demande de brevet WO 2004/089935 décrit également la préparation d'une nouvelle forme cristalline du sel de magnésium de l'esoméprazole trihydrate, dénommée H1, caractérisée par son diagramme de diffraction X sur poudre.

La demande de brevet WO 2007/031845 décrit elle aussi la préparation d'un sel de magnésium d'esoméprazole trihydrate, sous deux formes cristallines polymorphes G1 et G2, par réaction du sel de sodium de l'esoméprazole avec une solution aqueuse de sulfate de magnésium.

De son côté, la demande de brevet WO 98/54171 décrit la formation du sel de magnésium des énantiomères de l'oméprazole trihydrate, ainsi que du dihydrate sous les formes cristallines A et B. Il est indiqué que le trihydrate du sel de magnésium de l'esoméprazole peut être préparé par hydratation d'un sel de magnésium d'esoméprazole de forme cristalline I, obtenu comme décrit dans le document WO 96/01623, c'est-à-dire à partir d'esoméprazole et de méthoxyde de magnésium dans l'acétone. En variante, le trihydrate peut être préparé par réaction du sel de potassium de l'esoméprazole avec du sulfate de magnésium dans l'eau. Pour obtenir le dihydrate forme A, le sulfate de magnésium est dissous dans le méthanol, puis on ajoute un mélange d'eau et d'acétone au produit de la réaction.

Le document WO 2008/102145 enseigne un procédé alternatif de cristallisation du dihydrate forme A, censé pallier les insuffisances du procédé décrit dans WO 98/54171, qui conduirait à un composé instable, susceptible de se convertir au séchage en trihydrate ou en une forme amorphe. Les Exemples 7 à 9 de WO 2008/102145 divulguent ainsi un procédé comprenant des étapes de : (a) réaction du sel de potassium d'esoméprazole avec un sel de magnésium (tel que le chlorure de magnésium hexahydrate) en milieu alcoolique (notamment méthanolique), (b) filtration des insolubles, (c) ajout d'un mélange eau /acétone aux insolubles, (d) filtration, lavage à l'acétate d'éthyle et séchage.

Un tel procédé requiert l'utilisation de plusieurs solvants organiques dont certains, tels que le méthanol, peuvent poser des problèmes toxicologiques, et qui doivent en tout état de cause être éliminés au cours du procédé pour obtenir un produit le plus pur possible.

Plus récemment, il a été proposé dans la demande FR 2 920 428 un procédé décrit comme conduisant au sel de magnésium d'esoméprazole tétrahydrate. Ce procédé, illustré à l'Exemple 10 de cette demande, consiste à dissoudre dans une solution aqueuse de potasse un sel de potassium d'esoméprazole, puis à faire réagir ce sel avec du chlorure de magnésium dissous dans un mélange d'eau et d'acétone. Il est indiqué que le tétrahydrate est obtenu avec un rendement non optimisé de 35%. Il est toutefois apparu aux inventeurs, après de multiples tentatives, que ce procédé n'était pas reproductible et conduisait pratiquement à chaque fois au trihydrate du sel de magnésium d'esoméprazole.

La description générale de ce document suggère d'autres procédés de synthèse du tétrahydrate par échange de cations à partir du sel de potassium, sans plus de précision. Or, l'enseignement de l'art antérieur cité précédemment montre que la réaction d'un sel de magnésium sur le même sel de potassium d'esoméprazole, en fonction notamment des solvants utilisés, conduit à une diversité de formes cristallines et/ou d'hydrates. En outre, la nécessité de conduire la réaction en présence de potasse induit une pollution du produit obtenu par des ions potassium résiduels, qui affecte la pureté de ce produit.

Il serait donc souhaitable de pouvoir disposer d'un nouveau procédé de préparation d'un sel de magnésium d'énantiomère de l'oméprazole tétrahydraté qui soit économique, aisé à mettre en oeuvre, et qui conduise de façon reproductible et avec un rendement d'au moins 50% à un composé présentant une pureté acceptable pour une application pharmaceutique, notamment une faible teneur en potassium.

Dans ce contexte, les inventeurs ont mis au point un procédé de préparation du sel de magnésium de (S) oméprazole tétrahydrate satisfaisant à ce besoin. Ce procédé peut être aisément transposé à la préparation du distomère correspondant. De façon tout-à-fait surprenante, un tel procédé conduit de façon reproductible au tétrahydrate de ce sel, alors que l'acétone est plutôt connue pour son effet déshydratant, de sorte que l'on aurait pu s'attendre à obtenir le trihydrate, voire le dihydrate de ce sel.

Les inventeurs ont par ailleurs démontré la formation, dans ce procédé de préparation, d'une phase précurseur du tétrahydrate du sel de magnésium de l'énantiomère, qui conditionne l'obtention de ce tétrahydrate et n'a encore jamais été mise en évidence à leur connaissance.

La présente invention a ainsi pour objet une phase précurseur du sel de magnésium tétrahydraté d'un énantiomère de l'oméprazole (ci-après, "phase précurseur"), caractérisée en ce que son diffractogramme aux rayons X présente les pics caractéristiques suivants :

| d (Angstrom) | 2-Theta (°) | Intensité relative (%) |
|---|---|---|
| 19,80 | 4,45 | très forte |
| 12,17 | 7,25 | faible |
| 10,78 | 8,19 | moyenne |
| 9,26 | 9,53 | faible |
| 7,28 | 12,14 | faible |
| 6,79 | 13,03 | faible |
| 5,22 | 16,95 | faible |
| 5,01 | 17,67 | moyenne |
| 4,821 | 18,38 | faible |
| 4,009 | 22,15 | faible |

Elle a également pour objet trois procédés de préparation de ladite phase précurseur.

Le premier de ces procédés comprend le ré-empâtage d'un sel de magnésium dudit énantiomère sous forme amorphe, dans un mélange solvant / eau, ledit solvant étant choisi parmi les solvants organiques non alcooliques miscibles à l'eau et le rapport volumique du solvant à l'eau étant compris entre 95:5 et 30:70.

Le second procédé comprend :
- la mise en solution d'un sel alcalin éventuellement solvaté dudit énantiomère d'oméprazole dans un mélange solvant / eau, ledit solvant étant choisi parmi les solvants organiques non alcooliques miscibles à l'eau et le rapport volumique du solvant à l'eau étant compris entre 90:10 et 50:50,
- le mélange de ladite solution de sel alcalin avec une solution aqueuse de sel de magnésium dépourvue de solvant organique, pour former une suspension.

Le troisième procédé comprend :
- le ré-empâtage dans au moins un solvant organique non alcoolique miscible à l'eau, à l'exclusion de tout autre solvant, d'un sel de magnésium de l'énantiomère d'oméprazole, amorphe ou cristallin, éventuellement hydraté ou solvaté,
- l'ajout d'eau au produit ainsi obtenu.

La présente invention a en outre pour objet un procédé de préparation d'un sel de magnésium tétrahydraté d'un énantiomère de l'oméprazole, comprenant la filtration, puis le séchage, d'une suspension contenant la phase précurseur précitée.

Elle a encore pour objet des cristaux de sel de magnésium tétrahydraté d'un énantiomère de l'oméprazole obtenus suivant ce procédé.

Elle a par ailleurs pour objet ces cristaux pour une utilisation comme médicament, notamment dans la prévention ou le traitement des ulcères gastriques et/ou duodénaux.

Elle a enfin pour objet l'utilisation de ces cristaux pour la préparation du sel de magnésium d'un énantiomère d'oméprazole sous forme de dihydrate A, ainsi qu'un procédé de préparation du sel de magnésium d'un énantiomère d'oméprazole sous forme de dihydrate A, caractérisé en ce qu'il comprend une étape de déshydratation ménagée de cristaux du sel de magnésium tétrahydraté d'un énantiomère d'oméprazole.

Comme indiqué ci-dessus, l'invention vise à proposer des procédés permettant d'aboutir à la formation d'une nouvelle phase précurseur donnant elle-même naissance, de façon simple et reproductible, au sel de magnésium d'un énantiomère d'oméprazole sous forme de tétrahydrate (parfois désigné ci-après, pour plus de simplicité, par "tétrahydrate"), qui peut lui-même conduire directement au sel de magnésium de l'énantiomère sous forme de dihydrate A (parfois désigné ci-après, pour plus de simplicité, par "dihydrate A").

Cette phase précurseur, qui a été caractérisée par diffractométrie de rayons X sur poudre, constitue donc une nouvelle voie d'accès, non seulement au tétrahydrate mais également au dihydrate A, à partir d'un énantiomère d'oméprazole sous forme cristalline ou amorphe, éventuellement hydratée et/ou solvatée. Le rendement de ces procédés est généralement supérieur à 50%, notamment supérieur à 60%, voire supérieur à 70%, ce dernier étant calculé comme le rapport molaire du sel de magnésium (tétrahydrate ou dihydrate A) isolé à l'énantiomère mis en oeuvre.

Les voies d'accès à cette phase précurseur seront à présent décrites.

Dans cette description, l'expression "compris entre" doit s'entendre comme incluant les bornes citées. En outre, toutes les valeurs de l'angle de diffraction 2θ indiquées dans cette description s'entendent à 0,2° près et doivent être comprise comme mesurées par diffraction de rayons X sur poudre à l'aide d'un tube à anticathode de cuivre.

### Préparation du précurseur à partir d'un composé amorphe

Dans une première forme d'exécution de l'invention, la phase précurseur précitée est obtenue à partir d'un sel de magnésium d'un énantiomère de l'oméprazole sous forme amorphe.

Le sel de magnésium de cet énantiomère peut notamment être préparé suivant un procédé comprenant :
a) la réaction d'un sel alcalin éventuellement solvaté dudit énantiomère avec une solution aqueuse de sel de magnésium dépourvue de solvant organique, pour obtenir un précipité,
b) la filtration dudit précipité,
c) éventuellement, le ré-empâtage du résidu issu de l'étape (b) dans l'eau, suivi d'une filtration,
d) éventuellement, le séchage de la suspension ainsi obtenue.

Le sel alcalin de l'énantiomère mis en oeuvre dans l'étape (a) est avantageusement obtenu suivant un procédé de cristallisation préférentielle comprenant : (a1) la transformation de l'Oméprazole racémique en son sel de potassium sous forme de solvate, par ajout d'un excès de base minérale (telle que la potasse) et d'un solvant (tel que l'éthanol et/ou l'éthylèneglycol), pour obtenir un conglomérat, (a2) la préparation d'un mélange de cristaux dudit conglomérat, d'un premier énantiomère de l'oméprazole et d'un solvant, (a3) le refroidissement du mélange selon une certaine cinétique de température, et sous une agitation croissante, pour favoriser la croissance dudit énantiomère tout en évitant la nucléation de l'autre énantiomère, et (a4) la récupération des cristaux du premier énantiomère. Le conglomérat est ensuite ajouté en masse égale à la récolte aux liqueurs mères, pour obtenir un mélange biphasé, à partir duquel le second énantiomère est cristallisé à son tour par refroidissement. Ce procédé, dit AS3PC (cristallisation préférentielle polythermique programmée et auto-ensemencée), est décrit dans la demande FR 2 920 428, dont l'enseignement est incorporé ici par référence.

De préférence, le sel alcalin dudit énantiomère est le sel de potassium du (S) oméprazole éventuellement solvaté par l'éthanol ou l'éthylène glycol. Plus préférentiellement, il s'agit du sel de potassium du (S) oméprazole solvate d'éthanol.

Ce sel alcalin est avantageusement solubilisé dans l'eau (généralement en l'absence de tout autre solvant ou sel), par exemple à raison de 0,1 à 0,5 g/ml, voire de 0,2 à 0,3 g/ml, avant d'être ensuite mis à réagir avec une solution aqueuse de sel de magnésium dépourvue de solvant organique, pour obtenir un précipité. La concentration en magnésium de la solution aqueuse peut par exemple aller de 0,05 à 0,5 g/ml, notamment de 0,1 à 0,2 g/ml. On préfère par ailleurs utiliser de 0,1 à 1,5 équivalent, plus préférentiellement de 0,5 à 1 équivalent et, mieux, de 0,51 à 0,55 équivalent (en mole) de sel de magnésium par rapport au sel alcalin d'énantiomère de l'oméprazole.

Des exemples de sels de magnésium utilisables sont l'acétate, le chlorure, le bromure et le sulfate de magnésium. On préfère dans cette étape que la solution de sel de magnésium soit ajoutée à la solution de sel d'énantiomère d'oméprazole, plutôt que l'inverse.

En outre, la réaction est généralement effectuée sous agitation, pendant une durée de 30 secondes à 30 minutes, à une température allant de 0 à 30°C, de préférence de 5 à 20°C, mieux, d'environ 10°C.

Le composé obtenu peut être ré-empâté dans l'eau, en particulier si l'on souhaite réduire la teneur en potassium résiduel qu'il renferme, de façon à ce qu'elle soit inférieure à 1.000 ppm, de préférence inférieure ou égale à 500 ppm (telle que mesurée par spectrométrie d'émission de flamme). Après séchage éventuel dans des conditions douces, on obtient un sel de magnésium d'énantiomère d'oméprazole sous forme amorphe.

En variante, ce sel amorphe peut être préparé à partir d'un sel de magnésium d'un énantiomère d'oméprazole sous forme d'un hydrate quelconque, suivant un procédé comprenant les étapes de : (a) dissolution dudit sel dans un solvant approprié, (b) filtration dans des conditions permettant l'élimination des germes de cristallisation, par exemple sur membrane de 0,23 µm, (c) évaporation rapide ou lyophilisation en vue d'obtenir un précipité contenant le sel sous forme amorphe.

D'autres procédés de préparation d'un sel de magnésium d'énantiomère d'oméprazole sous forme amorphe sont décrits dans le document WO 2006/096709 et pourront être mis en oeuvre par l'homme du métier. Ils comprennent l'élimination rapide d'un solvant (en particulier par évaporation rapide) d'une solution renfermant un sel de magnésium d'énantiomère d'oméprazole. Ce dernier peut notamment être obtenu par réaction de magnésium dans un solvant, tel que le méthanol, avec un hydrocarbure chloré, tel que le dichlorométhane, pour obtenir un alcoxyde de magnésium, qui est ensuite mis à réagir avec l'énantiomère d'oméprazole.

En variante, le sel de magnésium de l'énantiomère sous forme amorphe peut être obtenu par atomisation d'une solution alcoolique d'un sel de magnésium dudit énantiomère sous la forme de l'un quelconque de ses hydrates.

Le sel de magnésium d'énantiomère d'oméprazole sous forme amorphe peut être converti en la phase précurseur recherchée suivant un procédé comprenant une première étape de ré-empâtage du composé amorphe dans un mélange solvant/eau, sous réserve de choisir un solvant organique polaire non alcoolique, et de respecter une proportion volumique correcte de solvant par rapport à l'eau. Le solvant est miscible à l'eau, c'est-à-dire au moins partiellement miscible à l'eau et de préférence miscible à l'eau en toutes proportions. Ce solvant est de préférence une cétone telle que l'acétone. En variante, on peut toutefois utiliser l'acétonitrile, le THF, la méthyléthyl cétone ou les mélanges, en toutes proportions, de ces solvants entre eux et/ou avec l'acétone.

Il a en effet été mis en évidence qu'à une température donnée, le recours à une proportion d'eau trop élevée pouvait conduire à un sel d'énantiomère d'oméprazole sous forme de trihydrate, même en effectuant rapidement la filtration ultérieure. Inversement, une proportion de solvant trop élevée affecte négativement le rendement, ainsi que la pureté du dihydrate forme A susceptible d'être obtenu ultérieurement. La proportion volumique de l'acétone à l'eau pourra être ajustée entre 95:5 et 30:70 par l'homme du métier, en fonction de la température de travail et du rapport massique du soluté (sel amorphe) au mélange de solvants (eau et acétone). On préfère que le rapport volumique de l'acétone à l'eau soit compris entre 75:25 et 40:60, plus préférentiellement entre 70:30 et 50:50, en particulier dans le cas où l'étape de ré-empâtage est effectuée à température ambiante (23°C). Pour le calcul du rapport volumique du solvant à l'eau, il convient de prendre en compte la quantité d'eau éventuellement combinée au sel amorphe, dans le cas où ce dernier n'a pas été soumis à une étape de séchage. En tout état de cause, on préfère ne pas utiliser dans cette étape d'autres solvants ou sels que ceux mentionnés ci-dessus.

L'étape de ré-empâtage est généralement mise en oeuvre sous agitation. La durée d'agitation peut être comprise entre 30 minutes et 15h, par exemple entre 1h et 12h, en particulier entre 4h et 12h. Celle-ci peut varier en fonction des paramètres du procédé (rapport volumique solvant/eau et température de travail) et des caractéristiques recherchées pour le procédé (notamment sa productivité) et/ou le produit (en particulier sa cristallinité).

La température à laquelle est effectué le ré-empâtage est généralement maintenue entre -10 et 25°C, de préférence entre 0 et 15°C, plus préférentiellement à environ 10°C. Il a été observé qu'une température plus basse permettait de réduire le ratio acétone/eau et d'augmenter ainsi le rendement en tétrahydrate.

Cette étape de ré-empâtage conduit à la phase précurseur décrite précédemment.

### Préparation du précurseur à partir d'un sel alcalin de l'énantiomère

Dans une seconde forme d'exécution de l'invention, la phase précurseur recherchée peut également être obtenue à partir d'un sel alcalin éventuellement solvaté de l'énantiomère d'oméprazole correspondant, en inversant les étapes d'échange de cation et de ré-empâtage décrites précédemment.

La première étape de ce procédé comprend la mise en solution, dans un mélange solvant / eau, dudit sel alcalin éventuellement solvaté de l'énantiomère d'oméprazole. La nature du solvant et les conditions de mise en oeuvre de cette étape peuvent être choisis, et varier dans la même mesure, que dans la première forme d'exécution de l'invention. Toutefois, dans cette forme d'exécution, on préfère que le rapport volumique du solvant à l'eau soit compris entre 90:10 et 50:50, par exemple entre 80:20 et 60:40. Par ailleurs, on préfère utiliser de 3 à 10 litres de mélange de solvants, avantageusement de 5 à 7 litres de mélange de solvants, par kilogramme de sel alcalin mis en oeuvre. En tout état de cause, on préfère ne pas utiliser dans cette étape d'autres solvants ou sels que ceux mentionnés ci-dessus.

Dans la seconde étape du procédé selon la deuxième forme d'exécution de l'invention, on mélange la solution du sel alcalin décrite ci-dessus avec une solution aqueuse de sel de magnésium dépourvue de solvant organique, pour former une suspension contenant la phase précurseur recherchée. On préfère dans cette étape que le sel de magnésium soit introduit dans la solution de sel alcalin, bien que l'inverse soit également envisageable.

Là encore, la nature et la quantité de sel de magnésium, ainsi que les paramètres de procédé de cette étape, peuvent être identiques à ceux décrits précédemment par référence à la première forme d'exécution de l'invention. On préfère toutefois que la durée de la réaction entre le sel alcalin et le sel de magnésium soit comprise entre 30 secondes et 4 heures, par exemple entre 2 et 3 heures, bornes incluses. Il est ensuite possible de maintenir la suspension obtenue sous agitation pendant quelques heures, par exemple jusqu'à 4 heures.

Dans une troisième forme d'exécution de l'invention, la phase précurseur recherchée peut également être obtenue à partir d'un sel de magnésium d'un énantiomère de l'oméprazole. Ce dernier peut se trouver sous une forme amorphe ou cristalline quelconque, éventuellement sous forme d'hydrate (dihydrate A ou B, trihydrate...) ou de solvate.

La première étape de ce procédé consiste en un ré-empâtage du sel de magnésium de l'énantiomère dans au moins un solvant organique miscible à l'eau (c'est-à-dire un solvant organique polaire au moins partiellement miscible à l'eau et de préférence miscible à l'eau en toutes proportions), à l'exclusion de tout autre solvant. Des exemples de tels solvants comprennent l'acétone, le THF, la méthyléthyl cétone et leurs mélanges. Cette étape permet simultanément la dissolution dudit sel et la cristallisation d'un produit sous la forme d'une phase efflorescente. Dans le cas où le solvant est l'acétone, cette phase est caractérisée en ce que son diffractogramme aux rayons X présente les pics caractéristiques suivants :

| d (Angstrom) | 2-Theta (°) | Intensité |
|---|---|---|
| 15,27 | 5,78 | très forte |
| 12,41 | 7,12 | faible |
| 8,52 | 10,37 | faible |
| 7,61 | 11,61 | faible |
| 6,79 | 13,03 | très faible |
| 5,10 | 17,38 | moyenne |

La température à laquelle est effectuée le ré-empâtage est généralement maintenue entre -10 et 30°C, de préférence entre 15 et 25°C, plus préférentiellement à température ambiante. Il a été observé qu'une température plus basse diminuait la cinétique de conversion. Par ailleurs, le rapport en poids/volume du sel d'énantiomère à l'acétone est généralement compris entre 1:7 à 1:20 (g/ml), par exemple d'environ 1:10 (g/ml).

Le principe de la seconde étape de ce procédé consiste en la conversion dudit produit en la phase précurseur du tétrahydrate par ajout d'eau. Cette étape est généralement réalisée à une température comprise entre -10 et 30°C, de préférence entre 0 et 10°C. En outre, le rapport volumique de l'eau au solvant organique (de préférence l'acétone) mis en oeuvre dans la seconde étape est généralement compris entre 5:95 et 60:40, pour une température voisine de 10°C, voire entre 3:97 et 70:30, pour une température proche de 0°C.

### Préparation et utilisations du tétrahydrate

Il a été observé que la phase précurseur obtenue suivant l'une des formes d'exécution précitées de l'invention donnait naissance, de façon surprenante, par simple filtration du milieu la contenant, au sel de magnésium de l'énantiomère d'oméprazole sous forme de tétrahydrate.

Cette étape de filtration de la suspension peut être réalisée au moyen d'un filtre ouvert, d'un filtre clos, d'un filtre sécheur ou d'une essoreuse. On préfère qu'elle soit mise en oeuvre sur verre fritté ou par centrifugation. L'étape de filtration peut éventuellement être suivie d'un rinçage et d'un séchage du produit ou "gâteau" obtenu, dans des conditions douces.

Le rinçage peut être effectué à l'eau ou à l'aide d'un solvant organique polaire tel que l'acétone. Le choix du solvant influe sur le rendement du procédé et la cristallinité du produit obtenu. Cette dernière est améliorée dans le cas où on utilise l'acétone, au détriment du rendement qui est meilleur dans le cas où on utilise l'eau. En variante, il est possible de rincer le tétrahydrate successivement avec de l'eau puis avec de l'acétone ou avec un mélange eau/acétone. En particulier, il a été mis en évidence qu'un rinçage final à l'acétone permettait d'obtenir ultérieurement le dihydrate A avec une plus grande pureté. Le rinçage peut être effectué par percolation de ces solvants au travers du tétrahydrate, ou par empâtage rapide du tétrahydrate dans ces solvants, suivi d'une étape finale de filtration.

L'étape de séchage est quant à elle généralement mise en oeuvre dans des conditions douces (température de 20 à 25°C, pression atmosphérique, sous flux d'air non sec) pour éviter de dégrader le tétrahydrate, qui constitue une phase métastable.

Le tétrahydrate obtenu n'est toutefois pas instable dans des conditions ambiantes de température, de pression et d'humidité relative. Il peut être caractérisé par son diffractogramme de rayons X sur poudre, comme indiqué à l'Exemple 3 ci-après.

Ce composé peut être utilisé comme médicament, notamment dans la prévention ou le traitement des ulcères gastriques et/ou duodénaux. Dans cette optique, il peut être véhiculé dans tout type de composition solide, par exemple sous forme de gélules, de comprimés, de capsules molles, de granules ou de poudre à diluer extemporanément, pour fabriquer un médicament adapté à être administré par voie orale. Ces compositions peuvent comprendre des excipients pharmaceutiquement acceptables, que l'homme du métier pourra aisément choisir en fonction de la forme galénique retenue, et éventuellement un ou plusieurs autres actifs thérapeutiques. La quantité de tétrahydrate incluse dans ces compositions est fonction de la dose quotidienne administrée, qui peut elle-même varier entre 5 et 300 mg, par exemple, ainsi que du nombre de prises quotidiennes prévues.

En variante, il peut être utilisé pour la préparation du sel de magnésium d'un énantiomère d'oméprazole sous forme de dihydrate A.

La présente invention porte donc également sur ces utilisations et en particulier sur un procédé de préparation du sel de magnésium d'un énantiomère d'oméprazole sous forme de dihydrate A, caractérisé en ce qu'il comprend une étape de déshydratation ménagée de cristaux du sel de magnésium tétrahydraté d'un énantiomère d'oméprazole susceptible d'être obtenu comme décrit précédemment.

Cette étape de déshydratation ménagée peut comprendre un séchage :
- à une température comprise entre 40 et 65°C, sous une pression de 1 Bar ou avantageusement sous vide, éventuellement à 0% d'humidité relative, ou
- à une température comprise entre 20 et 40°C, par exemple à température ambiante (23°C), sous vide et/ou sous 0% d'humidité relative (par exemple sous P₂O₅ ou sous flux d'azote sec).

Pour obtenir un produit plus pur, on préfère selon l'invention que le produit soit séché sous vide (compris entre 1 et 100 millibars, préférentiellement entre 20 et 80 millibars, mieux, entre 40 et 60 millibars), à une température comprise entre 20 et 40°C (avantageusement voisine de 30°C) pendant une durée allant de 10 heures à 3 jours, éventuellement en présence d'un léger débit d'azote.

D'autres avantages et caractéristiques de l'invention ressortiront des exemples qui suivent et dans lesquels il sera fait références aux figures en annexe où :
- la figure 1 montre le diffractogramme du sel de magnésium de l'esoméprazole amorphe ;
- la figure 2 montre le diffractogramme du tétrahydrate du sel de magnésium de l'esoméprazole ;
- la figure 3 montre la TGA-DSC du tétrahydrate du sel de magnésium de l'esoméprazole ;
- la figure 4 montre le diffractogramme de la phase précurseur du tétrahydrate ;
- la figure 5 illustre le diffractogramme du dihydrate forme A du sel de magnésium de l'esoméprazole ;

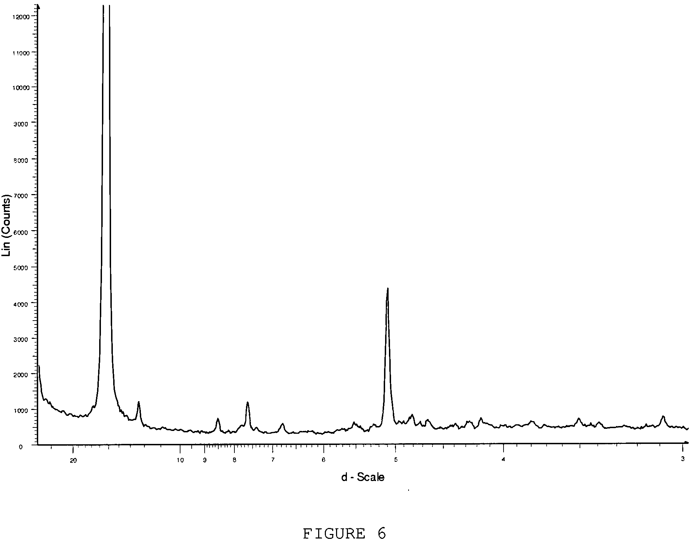

- la figure 6 illustre le diffractogramme de la phase efflorescente conduisant à la phase précurseur du tétrahydrate.

### EXEMPLES

### Méthodes analytiques utilisées

### Détermination de l'excès énantiomérique (% ee)

Les excès énantiomériques sont déterminés par chromatographie HPLC chirale à l'aide d'une colonne ChiralPAK AD (dimension 250 mm x 4,6 mm). Les conditions expérimentales sont :

| | |
|---|---|
| - Solvant : | 100% Ethanol absolu |
| - Débit : | 1 ml.min⁻¹ |
| - Détecteur : | λ = 302 nm |
| - Injection : | 20 µL |
| - Concentration : | environ 0,4 g.l⁻¹ dans l'éthanol. |

### Analyses par diffraction des RX sur poudre

Les analyses de diffraction des rayons X sur poudre (XRPD) ont été effectuées à l'aide d'un diffractomètre Bruker D8 équipé d'un détecteur Lynx Eye dans les conditions suivantes :
- Anticathode cuivre, voltage 40 kV, intensité 40mA
- Température ambiante
- Domaine de mesures : 3° à 30°
- Incrémentation entre chaque mesure : 0,04°
- Temps de mesure par pas : 1,5 s.

### Mesures calorimétriques et thermogravimétriques

Les analyses de TGA-DSC ont été réalisées à partir d'une TGA-DSC Netzsch STA 449C. Les mesures sont effectuées dans un creuset en aluminium, entre 0° et 200°C avec une vitesse d'échauffement de 2°C.min⁻¹ sous un balayage d'hélium de 60 ml.min⁻¹.

### Exemple 1 : Préparation du sel de magnésium de l'esoméprazole amorphe

Le sel de potassium de l'esoméprazole solvate d'éthanol, obtenu suivant le procédé décrit dans le brevet WO 2009/027614, est dissout (10g) dans l'eau (40 ml). Une solution aqueuse d'acétate de magnésium (10 ml, 37g.l⁻¹ en Mg²⁺) , est ajoutée à la solution précédente au goutte-à-goutte, sous agitation. Le précipité blanc obtenu est gardé sous agitation pendant 30 minutes puis filtré sur fritté N°3. Le précipité blanc ainsi récupéré est réempâté dans 40mL d'eau pendant 30 minutes, filtré sur fritté N°3, puis séché. Ce précipité blanc est un sel de magnésium de l'esoméprazole amorphe.

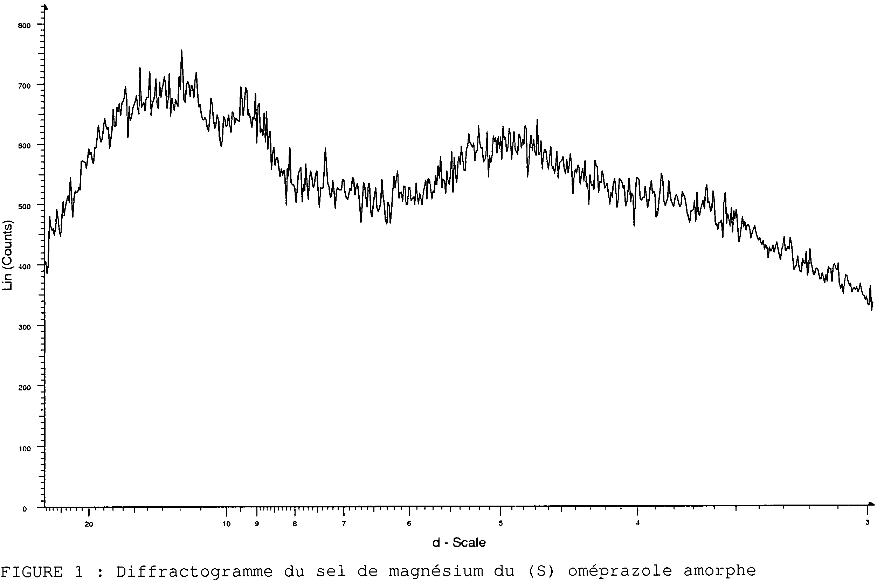

Le diffractogramme (DRXP) de ce composé est présenté sur la figure 1.

### Exemple 2 : Préparation du sel de magnésium du (S) Oméprazole amorphe

Le sel de potassium du (S) oméprazole solvate d'éthanol, obtenu suivant le procédé décrit dans le brevet WO 2009/027614, est dissout (10 g) dans l'eau (40 ml). Une solution aqueuse d'acétate de magnésium (20 ml, 19g.l⁻¹ en Mg²⁺) , est ajoutée à la solution précédente au goutte-à-goutte, sous agitation. Le précipité blanc obtenu est gardé sous agitation pendant 20 minutes puis filtré sur fritté N°3 puis séché. Ce précipité blanc est un sel de magnésium du (S) oméprazole amorphe.

### Exemple 3 : Préparation du sel de magnésium du (S) Oméprazole tétrahydrate (voie 1)

Le sel de magnésium du (S) oméprazole amorphe préparé à l'exemple 1 est ré-empâté dans un mélange acétone (45 ml) / eau (15 ml). La suspension est gardée sous agitation deux heures, filtrée puis séchée (à température et pression ambiante). Le solide blanc obtenu est le tétrahydrate du sel de magnésium du (S) Oméprazole. Ce composé a été obtenu avec un rendement de 62%.

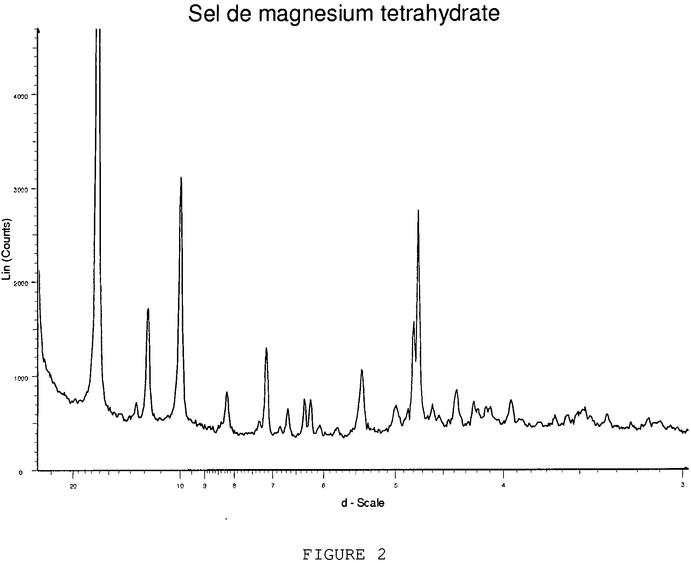

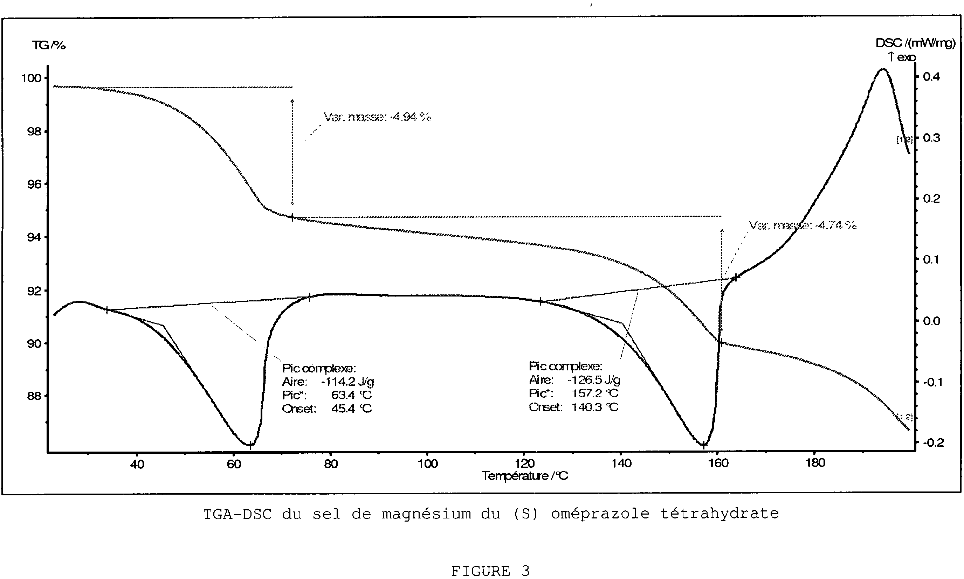

Le diffractogramme (DRXP) de ce composé est présenté sur la Figure 2 et sa TGA-DSC est illustrée à la Figure 3.

Le tableau I ci-dessous montre la position et l'intensité relative des pics caractéristiques du tétrahydrate du sel de magnésium de l'esoméprazole énantiomériquement pur (c'est-à-dire présentant une pureté e.e. > 99%).

**Tableau I**

| Sel de magnésium de l'esoméprazole tétrahydrate | | |
|---|---|---|
| Angle 2-Theta (2θ) | d (Angstrom) | Intensité I/Io % |
| 5,37 | 16,46 | 100 |
| 7,36 | 11,00 | 11,4 |
| 8,76 | 10,09 | 26,6 |
| 10,63 | 8,31 | 6,6 |
| 12,26 | 7,21 | 7,1 |
| 16,21 | 5,46 | 10,4 |
| 17,61 | 5,03 | 6,9 |
| 18,28 | 4,848 | 13 |
| 18,50 | 4,791 | 21,9 |

### Exemple 4 : Préparation du tétrahydrate du sel de magnésium du (S) Oméprazole (voie 1)

Le solide blanc obtenu à l'Exemple 2 est successivement réempâté dans 120 ml d'un mélange acétone / eau (75/25 %V/V) pendant 2 heures puis dans 60 ml d'un mélange acétone / eau (85/15 %V/V) pendant 1 heure. Après filtration et séchage, le tétrahydrate du sel de magnésium du (S) oméprazole est récupéré avec un rendement de 55%.

### Exemple 5 : Préparation du tétrahydrate du sel de magnésium du (S) Oméprazole (voie 1)

Le solide blanc obtenu à l'Exemple 2 est successivement réempâté dans 120 ml d'un mélange acétone / eau (75/25 %V/V) pendant 2 heures puis dans 60 ml d'un mélange acétone / eau (85/15 %V/V) pendant 1 heure. Après filtration et séchage, le tétrahydrate du sel de magnésium du (S) oméprazole est récupéré avec un rendement de 52%.

### Exemple 6 : Préparation du tétrahydrate du sel de magnésium du (S) Oméprazole (voie 1)

Cet exemple illustre un mode de préparation ne comportant pas d'étape de séchage du produit intermédiaire amorphe formé.

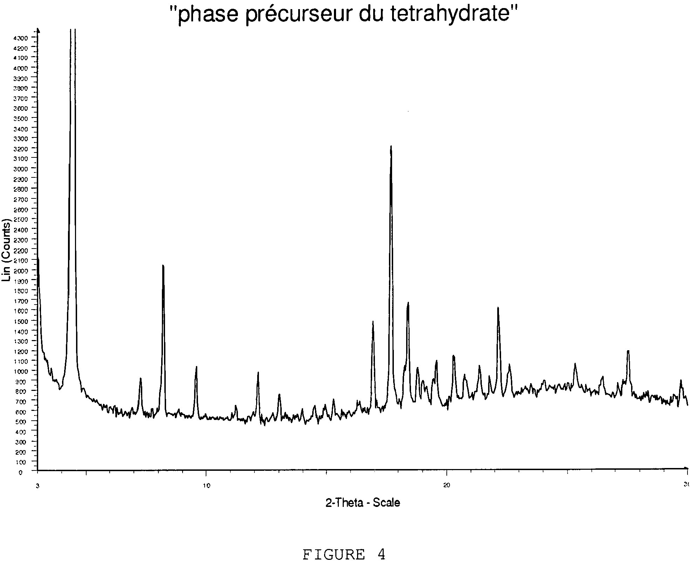

On a solubilisé 20g de sel de potassium de l'esoméprazole solvate d'éthanol, obtenu suivant le procédé décrit dans le brevet WO 2009/027614, dans 80 ml d'eau, à température ambiante. On a ajouté à cette solution 4,96g d'acétate de magnésium tétrahydrate dans 10 ml d'eau déminéralisée, sous agitation, puis 30 ml d'eau supplémentaires. Après quelques minutes d'agitation, le milieu a été essoré sur fritté. Le gâteau obtenu a été réempâté dans un mélange acétone/eau renfermant 55 ml d'acétone et 5 ml d'eau, soit un mélange à 50/50% en volume, en prenant en compte la quantité d'eau incluse dans le gâteau (40 ml). L'agitation a été poursuivie pendant 1 heure, après quoi on a mis en évidence la phase précurseur du tétrahydrate dans la suspension. Celle-ci a été caractérisée par DRX. Son diffractogramme est présenté sur la Figure 4 annexée.

Puis le gâteau a été filtré sur fritté et lavé avec 20 ml d'eau. On a ainsi isolé le tétrahydrate du sel de magnésium du (S) oméprazole.

### Exemple 7 : Préparation du tétrahydrate du sel de magnésium du (S) Oméprazole (voie 2)

On a solubilisé 312,5 g de sel de potassium de l'esoméprazole solvate d'éthanol, obtenu suivant le procédé décrit dans le brevet WO 2009/027614, dans 625 ml d'eau, puis on a ajouté 1250 ml d'acétone. Le milieu a alors été agité et refroidi à 10°C et on y a introduit 71,85 g d'acétate de magnésium tétrahydrate dans 625 ml d'eau déminéralisée, sur une période de trois heures. Après une nuit d'agitation à 10 °C, on a mis en évidence la présence de la phase précurseur dans le milieu.

Celui-ci a alors été essoré sur fritté, puis le gâteau a été lavé par deux lavages à l'eau déminéralisée (2 x 625 ml), suivis de deux lavages à l'acétone (2 x 625 ml). Le produit isolé et séché correspondait au sel de magnésium d'esoméprazole tétrahydrate.

### Exemple 8 : Préparation du tétrahydrate du sel de magnésium du (S) Oméprazole (voie 3)

1 g de sel de magnésium trihydraté a été ré-empâté pendant une nuit dans 10 mL d'acétone à température ambiante. La suspension a ensuite été refroidie à 10°C, puis 10 mL d'eau ont été ajoutés. Après 3 heures d'agitation à 10°C, le milieu a été filtré sur fritté et rincé avec 20 mL d'acétone. Le produit, correspondant au sel de magnésium tétrahydrate, a été isolé avec un rendement de 77%.

### Exemple 9 : Préparation du tétrahydrate du sel de magnésium du (S) Oméprazole (voie 3)

1 g de sel de magnésium trihydraté a été ré-empâté pendant une heure dans 10 mL d'acétone à température ambiante. La suspension a ensuite été refroidie à 10°C, puis 0,8 mL d'eau ont été ajoutés. Après 3 heures d'agitation à 10°C, le milieu a été filtré sur fritté et rincé avec 20 mL d'acétone. Le produit, correspondant au sel de magnésium tétrahydrate, a été isolé avec un rendement de 84%.

### Exemple (Comparatif) 10 :

Du sel de potassium de l'esoméprazole est dissous (7g) dans l'eau avec un excès de potasse (0,8g soit 0,2 équivalent molaire). On ajoute du chlorure de magnésium en excès (22,8g soit environ 5 équivalents molaires), préalablement dissous dans une solution eau/acétone (50 ml/100 ml). Cette solution est versée au goutte-à-goutte sous agitation. Le solide est récupéré par filtration sur Büchner, puis rincé abondamment à l'eau distillée.

Ce solide ne correspond pas au tétrahydrate du sel de magnésium du (S) oméprazole. Il est probable qu'il s'agisse du trihydrate correspond, qui est plus stable.

### Exemple 11 : Préparation du sel de magnésium du (S) Oméprazole dihydrate forme A

Le tétrahydrate obtenu à l'exemple 2 est séché sous 0% d'humidité relative, à 40°C. Le solide récupéré est le (S) oméprazole dihydrate forme A avec un rendement supérieur à 99%.

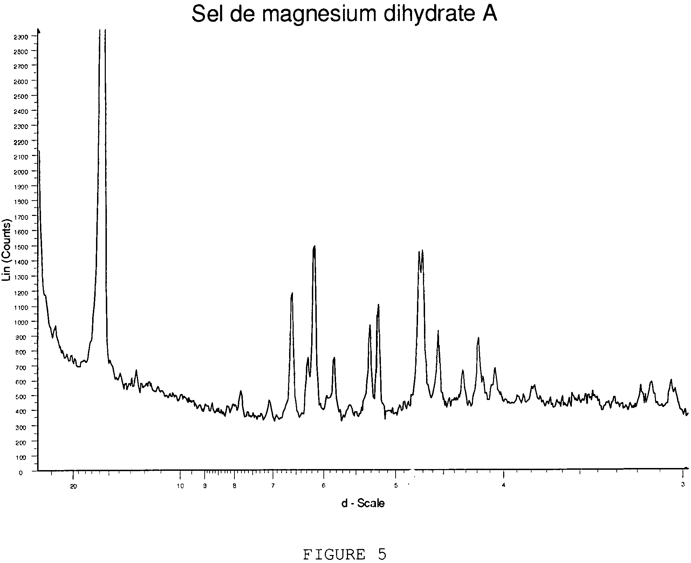

Le diffractogramme de rayons X sur poudre (DRXP) de cette phase est présenté sur la figure 5.

Le tableau II ci-dessous montre par ailleurs la position et l'intensité relative des pics caractéristiques pour le dihydrate forme A du sel de magnésium de l'esoméprazole énantiomériquement pur (c'est-à-dire présentant une pureté e.e. > 99%).

**Tableau II**

| Sel de magnésium de l'esoméprazole dihydrate forme A | | |
|---|---|---|
| Angle 2-Theta (2θ) | d (Angstrom) | Intensité I/Io % |
| 5,52 | 16,0 | 95,3 |
| 13,31 | 6,65 | 52,9 |
| 13,92 | 6,36 | 38,4 |
| 14,26 | 6,21 | 85,6 |
| 15,08 | 5,872 | 46,7 |
| 16,53 | 5,359 | 46,4 |
| 16,85 | 5,258 | 35,8 |
| 18,69 | 4,744 | 100 |
| 19,38 | 4,577 | 61,8 |
| 20,37 | 4,357 | 42 |
| 21,06 | 4,214 | 63,7 |
| 21,73 | 4,086 | 41,4 |
| 23,33 | 3,810 | 29,6 |
| 27,85 | 3,201 | 33,9 |
| 28,25 | 3,157 | 34,3 |
| 29,13 | 3,063 | 33,6 |

### Exemple 12 : Préparation du sel de magnésium du (S) Oméprazole dihydrate forme A

Le tétrahydrate obtenu à l'exemple 2 est séché à 60°C (de préférence sous 0% d'humidité relative). Le solide récupéré est le (S) oméprazole dihydrate forme A avec un rendement supérieur à 99%.

### Exemple 13 : Préparation du sel de magnésium du (S) Oméprazole dihydrate forme A

Le tétrahydrate obtenu à l'exemple 2 est séché sous 0% d'humidité relative, à 20°C et sous une pression de 100 mBar. Le solide récupéré est le du (S) oméprazole dihydrate forme A avec un rendement supérieur à 99%.

### Exemple 14 : Préparation du sel de magnésium du (S) Oméprazole dihydrate forme A

Le produit obtenu à l'Exemple 7 a été séché sous vide à 30°C, sous balayage d'azote. On a obtenu le dihydrate A avec un rendement de 80,9% par rapport au sel alcalin de l'énantiomère d'oméprazole mis en oeuvre à l'exemple 7.

### Exemple (Comparatif) 15 :

Le sel de magnésium du (S) oméprazole amorphe (0.5g), préparé à l'exemple 1, est réempâté dans l'acétone (10mL) pendant 2 heures, filtré puis séché (à température et pression ambiante). Le solide blanc obtenu est un dihydrate forme B du sel de magnésium du (S) Oméprazole, comme l'indique le diffractogramme (DRXP) de cette phase.

## Revendications

1. Phase précurseur du sel de magnésium tétrahydraté d'un énantiomère de l'oméprazole, **caractérisée en ce que** son diffractogramme aux rayons X présente les pics caractéristiques suivantes :
| d (Angstrom) | 2-Theta (°) | Intensité relative (%) |
|---|---|---|
| 19,80 | 4,45 | très forte |
| 12,17 | 7,25 | faible |
| 10, 78 | 8, 19 | moyenne |
| 9,26 | 9,53 | faible |
| 7,28 | 12, 14 | faible |
| 6, 79 | 13, 03 | faible |
| 5,22 | 16, 95 | faible |
| 5,01 | 17,67 | moyenne |
| 4, 821 | 18,38 | faible |
| 4,009 | 22, 15 | faible |

2. Procédé de préparation de la phase précurseur selon la revendication 1, **caractérisé en ce qu'**il comprend le ré-empâtage d'un sel de magnésium dudit énantiomère sous forme amorphe, dans un mélange solvant /eau, ledit solvant étant choisi parmi les solvants organiques non alcooliques miscibles à l'eau et le rapport volumique du solvant à l'eau étant compris entre 95:5 et 30:70.

3. Procédé selon la revendication 2, **caractérisé en ce que** le sel de magnésium dudit énantiomère sous forme amorphe est susceptible d'être obtenu suivant un procédé comprenant les étapes suivantes :
a) la réaction d'un sel alcalin éventuellement solvaté dudit énantiomère d'oméprazole avec une solution aqueuse de sel de magnésium dépourvue de solvant organique, pour obtenir un précipité,
b) la filtration dudit précipité,
c) éventuellement, le ré-empâtage du résidu issu de l'étape (b) dans l'eau, suivi d'une filtration,
d) éventuellement, le séchage de la suspension obtenue.

4. Procédé de préparation de la phase précurseur selon la revendication 1, **caractérisé en ce qu'**il comprend :
- la mise en solution d'un sel alcalin éventuellement solvaté dudit énantiomère d'oméprazole dans un mélange solvant / eau, ledit solvant étant choisi parmi les solvants organiques non alcooliques miscibles à l'eau et le rapport volumique du solvant à l'eau étant compris entre 90:10 et 50:50, et
- le mélange de ladite solution avec une solution aqueuse de sel de magnésium dépourvue de solvant organique, pour former une suspension.

5. Procédé de préparation de la phase précurseur selon la revendication 1, **caractérisé en ce qu'**il comprend :
- le ré-empâtage dans au moins un solvant organique non alcoolique miscible à l'eau, à l'exclusion de tout autre solvant, d'un sel de magnésium de l'énantiomère d'oméprazole, amorphe ou cristallin, éventuellement hydraté ou solvaté,
- l'ajout d'eau au produit ainsi obtenu.

6. Procédé selon l'une quelconque des revendications 2 à 4, **caractérisé en ce que** le solvant organique est choisi parmi : les cétones, telles que l'acétone et la méthyléthyl cétone ; l'acétonitrile ; le THF ; et leurs mélanges, de préférence l'acétone.

7. Procédé selon l'une quelconque des revendications 3 et 4, **caractérisé en ce que** le sel dudit énantiomère est le sel de potassium du (S) oméprazole éventuellement solvaté par l'éthanol ou l'éthylène glycol, de préférence le sel de potassium du (S) oméprazole solvate d'éthanol.

8. Procédé selon l'une quelconque des revendications 3 et 4, **caractérisé en ce que** le sel de magnésium est choisi parmi l'acétate, le sulfate, le bromure ou le chlorure de magnésium.

9. Procédé de préparation d'un sel de magnésium tétrahydraté d'un énantiomère de l'oméprazole, comprenant la filtration, puis le séchage, d'une suspension contenant la phase précurseur selon la revendication 1.

10. Cristaux de sel de magnésium tétrahydraté d'un énantiomère de l'oméprazole, obtenus suivant le procédé selon la revendication 9.

11. Cristaux selon la revendication 10 pour une utilisation comme médicament, notamment dans la prévention ou le traitement des ulcères gastriques et/ou duodénaux.

12. Utilisation des cristaux selon la revendication 10 pour la préparation du sel de magnésium d'un énantiomère d'oméprazole sous forme de dihydrate A.

13. Procédé de préparation du sel de magnésium d'un énantiomère d'oméprazole sous forme de dihydrate A, **caractérisé en ce qu'**il comprend une étape de déshydratation ménagée de cristaux du sel de magnésium tétrahydraté d'un énantiomère d'oméprazole selon la revendication 10.

14. Procédé selon la revendication 13, **caractérisé en ce que** la déshydratation ménagée comprend un séchage :
- à une température comprise entre 40 et 65°C, sous une pression de 1 Bar ou avantageusement sous vide, éventuellement à 0% d'humidité relative, ou
- à une température comprise entre 20 et 40°C, par exemple à température ambiante, sous vide et/ou sous 0% d'humidité relative.

## Claims

1. Precursor phase of the magnesium tetrahydrate salt of an omeprazole enantiomer, **characterized in that** its X-ray diffractogram has the following characteristic peaks:
| d (Angströms) | 2-Theta (°) | Relative intensity (%) |
|---|---|---|
| 19.80 | 4.45 | very strong |
| 12.17 | 7.25 | weak |
| 10.78 | 8.19 | medium |
| 9.26 | 9.53 | weak |
| 7.28 | 12.14 | weak |
| 6.79 | 13.03 | weak |
| 5.22 | 16.95 | weak |
| 5.01 | 17.67 | medium |
| 4.821 | 18.38 | weak |
| 4.009 | 22.15 | weak |

2. Process for preparing the precursor phase according to Claim 1, **characterized in that** it comprises the reslurrying of a magnesium salt of said enantiomer in amorphous form in a solvent/water mixture, said solvent being chosen from water-miscible nonalcoholic organic solvents and the volume ratio of the solvent to water being between 95/5 and 30/70.

3. Process according to Claim 2, **characterized in that** the magnesium salt of said enantiomer in amorphous form may be obtained according to a process comprising the following steps:
a) the reaction of an optionally solvated alkali metal salt of said omeprazole enantiomer with an aqueous solution of magnesium salt free of organic solvent, to obtain a precipitate,
b) the filtration of said precipitate,
c) optionally, the reslurrying of the residue obtained from step (b) in water, followed by filtration,
d) optionally, the drying of the suspension obtained.

4. Process for preparing the precursor phase according to Claim 1, **characterized in that** it comprises:
- the dissolution of an optionally solvated alkali metal salt of said omeprazole enantiomer in a solvent/water mixture, said solvent being chosen from water-miscible nonalcoholic organic solvents and the volume ratio of the solvent to water being between 90/10 and 50/50, and
- the mixing of said solution with an aqueous solution of a magnesium salt free of organic solvent, to form a suspension.

5. Process for preparing the precursor phase according to Claim 1, **characterized in that** it comprises:
- the reslurrying in at least one water-miscible nonalcoholic organic solvent, to the exclusion of any other solvent, of a magnesium salt of the amorphous or crystalline, optionally hydrated or solvated omeprazole enantiomer,
- the addition of water to the product thus obtained.

6. Process according to any one of Claims 2 to 4, **characterized in that** the organic solvent is chosen from: ketones, such as acetone and methyl ethyl ketone; acetonitrile; THF; and mixtures thereof, preferably acetone.

7. Process according to either of Claims 3 and 4, **characterized in that** the salt of said enantiomer is the potassium salt of (S) omeprazole optionally solvated with ethanol or ethylene glycol, preferably the potassium salt of (S) omeprazole solvated with ethanol.

8. Process according to either of Claims 3 and 4, **characterized in that** the magnesium salt is chosen from magnesium acetate, sulfate, bromide and chloride.

9. Process for preparing a magnesium tetrahydrate salt of an omeprazole enantiomer, comprising the filtration and then drying of a suspension containing the precursor phase according to Claim 1.

10. Crystals of the magnesium tetrahydrate salt of an omeprazole enantiomer, which is obtained according to the process according to Claim 9.

11. Crystals according to Claim 10, for use as medicament, especially in the prevention or treatment of gastric and/or duodenal ulcers.

12. Use of the crystals according to Claim 10 for the preparation of the magnesium salt of an omeprazole enantiomer in dihydrate form A.

13. Process for preparing the magnesium salt of an omeprazole enantiomer in dihydrate form A, **characterized in that** it comprises a step of controlled dehydration of crystals of the magnesium tetrahydrate salt of an omeprazole enantiomer according to Claim 10.

14. Process according to Claim 13, **characterized in that** the controlled dehydration includes drying:
- at a temperature of between 40 and 65°C, under a pressure of 1 bar or advantageously under vacuum, optionally at 0% relative humidity, or
- at a temperature of between 20 and 40°C, for example at room temperature, under vacuum and/or at 0% relative humidity.

## Patentansprüche

1. Ausgangsphase des Magnesiumtetrahydratsalzes von einem Enantiomer des Omeprazols, **dadurch gekennzeichnet dass** ihr Röntgen-Diffraktogramm die folgenden charakteristischen Peaks zeigt:
| d (Angstrom) | 2-Theta (°) | **relative Intensität %** |
|---|---|---|
| 19,80 | 4,45 | **sehr stark** |
| 12, 17 | 7,25 | **schwach** |
| 10, 78 | 8,19 | **mittel** |
| 9,26 | 9,53 | **schwach** |
| 7,28 | 12,14 | **schwach** |
| 6, 79 | 13,03 | **schwach** |
| 5,22 | 16,95 | **schwach** |
| 5,01 | 17,67 | **mittel** |
| 4,821 | 18,38 | **schwach** |
| 4,009 | 22,15 | **schwach** |

2. Verfahren zur Herstellung der Ausgangsphase nach Anspruch 1, **dadurch gekennzeichnet, dass** sie die Wiederaufschlämmung von einem Magnesiumsalz des Enantiomers in amorpher Form in einem Wasser / Lösungsmittel Gemisch umfasst, wobei das Lösungsmittel ausgewählt ist aus mit Wasser mischbaren nicht alkoholischen organischen Lösungsmitteln und das Volumenverhältnis des Lösungsmittels zu dem Wasser von 95:5 bis 30:70 beträgt.

3. Verfahren nach Anspruch 2, **dadurch gekennzeichnet, dass** das Magnesiumsalz des Enantiomers in amorpher Form erhaltbar ist durch ein Verfahren, das die folgenden Schritte umfasst:
a) Umsetzung eines gegebenenfalls solvatisierten Alkalisalzes des Omeprazol-Enantiomers mit einer wässrigen Lösung von Magnesiumsalz ohne organisches Lösungsmittel, um einen Niederschlag zu erhalten,
b) Filtration des Niederschlags,
c) gegebenenfalls Wiederaufschlämmung des Rückstands aus Schritt (b) in Wasser und anschließende Filtration,
d) gegebenenfalls Trocknung der erhaltenen Suspension.

4. Verfahren zur Herstellung der Ausgangsphase nach Anspruch 1, **dadurch gekennzeichnet, dass** es folgendes umfasst:
- Lösen eines alkalischen, gegebenenfalls solvatisierten Salz des Omeprazol-Enantiomers in einem Wasser / Lösungsmittel Gemisch, wobei das Lösungsmittel ausgewählt ist aus mit Wasser mischbaren nicht alkoholischen organischen Lösungsmitteln und das Volumenverhältnis des Lösungsmittels zu dem Wasser von 90:10 bis 50:50 beträgt, und
- Mischen der Lösung mit einer wässrigen Lösung von Magnesiumsalz ohne organisches Lösungsmittel, um eine Suspension zu bilden.

5. Verfahren zur Herstellung der Ausgangsphase nach Anspruch 1, **dadurch gekennzeichnet, dass** es folgendes umfasst:
- Wiederaufschlämmung in mindestens einem mit Wasser mischbaren nicht alkoholischen organischen Lösungsmittel, unter Ausschluss von sämtlichen anderen Lösungsmitteln, eines amorphen oder kristallinen, gegebenenfalls hydratisierten oder solvatisierten Magnesiumsalzes des Omeprazol-Enantiomers,
- Zugabe von Wasser zu dem so erhaltenen Produkt.

6. Verfahren nach einem der Ansprüche 2 bis 4, **dadurch gekennzeichnet, dass** das organische Lösungsmittel ausgewählt ist aus: Ketonen, wie Aceton und Methylethylketon, Acetonitril, THF und ihren Gemischen, vorzugsweise Aceton.

7. Verfahren nach einem der Ansprüche 3 und 4, **dadurch gekennzeichnet, dass** das Salz des Enantiomers das (S)-Omeprazol-Kaliumsalz ist, gegebenenfalls solvatisiert durch Ethanol oder Ethylenglycol, vorzugsweise das Kaliumsalz des (S)-Omeprazol-Ethanolsolvats.

8. Verfahren nach einem der Ansprüche 3 und 4, **dadurch gekennzeichnet, dass** das Magnesiumsalz ausgewählt ist aus Magnesiumacetat, -sulfat, -bromid, oder -chlorid.

9. Verfahren zur Herstellung von einem Magnesiumtetrahydratsalz von einem Enantiomer des Omeprazols, umfassend Filtration und anschließend Trocknung von einer Suspension, die die Ausgangsphase nach Anspruch 1 enthält.

10. Magnesiumtetrahydratsalzkristalle von einem Enantiomer des Omeprazols, erhalten durch das Verfahren nach Anspruch 9.

11. Kristalle nach Anspruch 10 für eine Verwendung als Medikament, insbesondere für die Prävention oder die Behandlung von Magen- und/oder Zwölffingerdarmgeschwüren.

12. Verwendung der Kristalle nach Anspruch 10 zur Herstellung des Magnesiumsalzes von einem Omeprazol-Enantiomer in Dihydrat A-Form.

13. Verfahren zur Herstellung des Magnesiumsalzes von einem Omeprazol-Enantiomer in Dihydrat A-Form, **dadurch gekennzeichnet, dass** es einen kontrollierten Deshydratationsschritt der Kristalle des Magnesiumtetrahydratsalzes von einem Omeprazol-Enantiomer nach Anspruch 10 umfasst.

14. Verfahren nach Anspruch 13, **dadurch gekennzeichnet, dass** die kontrollierte Deshydratation eine Trocknung umfasst:
- bei einer Temperatur von 40 bis 65 °C, unter einem Druck von 1 Bar oder vorteilhafterweise unter Vakuum, gegebenenfalls bei 0% relativer Feuchtigkeit, oder
- bei einer Temperatur von 20 bis 40 °C, beispielsweise Umgebungstemperatur, unter Vakuum und/oder unter 0% relativer Feuchtigkeit.
